(19) Europäisches Patentamt
European Patent Office
Office européen des brevets

(11) EP 1 224 172 B1

(12) # EUROPEAN PATENT SPECIFICATION

(45) Date of publication and mention of the grant of the patent:
**04.04.2007 Bulletin 2007/14**

(21) Application number: **00973329.6**

(22) Date of filing: **24.10.2000**

(51) Int Cl.:
***C07D 215/54*** *(2006.01)* ***C07D 215/56*** *(2006.01)*
***A61K 31/47*** *(2006.01)* ***A61K 31/4704*** *(2006.01)*
***A61K 31/4706*** *(2006.01)* ***A61P 35/00*** *(2006.01)*

(86) International application number:
**PCT/SE2000/002055**

(87) International publication number:
**WO 2001/030758 (03.05.2001 Gazette 2001/18)**

(54) **DRUGS FOR THE TREATMENT OF MALIGNANT TUMOURS**

DROGEN ZUR BEHANDLUNG MALIGNER TUMOREN

MEDICAMENTS DE TRAITEMENT DE TUMEURS MALIGNES

(84) Designated Contracting States:
**AT BE CH CY DE DK ES FI FR GB GR IE IT LI LU MC NL PT SE**
Designated Extension States:
**LT LV RO**

(30) Priority: **25.10.1999 SE 9903838**
**25.10.1999 US 161407 P**
**21.06.2000 SE 0002320**
**21.06.2000 US 212816 P**

(43) Date of publication of application:
**24.07.2002 Bulletin 2002/30**

(73) Proprietor: **Active Biotech AB**
**220 07 Lund (SE)**

(72) Inventors:
- **HEDLUND, Gunnar**
  **S-224 56 Lund (SE)**
- **JANSSON, Karl**
  **S-240 10 Dalby (SE)**
- **JÖNSSON, Stig**
  **S-222 41 Lund (SE)**
- **BJÖRK, Anders**
  **S-237 36 Bjärred (SE)**

(74) Representative: **Allee, Harriet Eva Charlotta et al**
**Dr Ludwig Brann Patentbyra AB,**
**Box 17192**
**104 62 Stockholm (SE)**

(56) References cited:

| | |
|---|---|
| **EP-A1- 0 059 698** | **WO-A1-00/03991** |
| **WO-A1-00/03992** | **WO-A1-92/18483** |
| **WO-A1-95/24395** | **WO-A1-99/55678** |
| **GB-A- 2 290 786** | **US-A- 6 121 287** |

- **R. DE WIT ET AL.: 'EORTC phase II study of daily oral linomide in metastatic renal cell carcinoma patients with good prognostic factors' EUROPEAN JOURNAL OF CANCER vol. 33, 1997, pages 493 - 495, XP002936443**

Remarks:
The file contains technical information submitted after the application was filed and not included in this specification

EP 1 224 172 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### FIELD OF THE INVENTION

The present invention relates to the use of quinoline derivatives for the manufacturing of a medicament for the clinical treatment of a plurality of malignant tumours, especially in solid form. Furthermore, the present invention relates to structurally novel quinoline derivatives, to methods for their preparation and to compositions containing them. The types of cancer that are especially inhibited by the quinoline derivatives of the present invention include, for example, breast cancers, colon cancers, Kaposi's sarcoma, lung cancers, ovarian cancers, prostatic cancers, and skin cancers. More particularly, the present invention relates to quinoline derivatives suitable for treatment of and preventing the development of prostatic cancer, and for preventing and treatment of the metastases of prostatic cancer.

### BACKGROUND OF THE INVENTION

Solid tumours, primary or metastases consist of several types of cells where the tumour cells are the pivotal cell type and the driving force. When microtumours reach a certain size, the requirement of nutrition cannot be satisfied by plain diffusion. New vessels penetrate the tumour establishing a microenvironment providing the tumour with optimal nutrition for further proliferation and growth. This tumour induced angiogenesis, inducing proliferation, migration and differentiation of normal endothelial cells from nearby small blood vessels, is a prerequisite for the growth of solid tumours. In concordance the inhibition of angiogenesis results in an effective inhibition of growth of solid tumours.

Solid tumours spread by penetrating tissue borders and give rise to daughter cell colonies or metastases. Single tumour cells or small cell aggregates spread by the blood or the lymph system to distant sites. In this process the tumour cells are vulnerable and can be extinguished by natural killer (NK) cells, a distinct type of cytotoxic lymphocytes. Enhanced activity or an increased number of NK cells reduce or inhibit the metastasising process in solid tumour disease.

Prostatic cancer is a malignant tumour disease that spreads to distant sites as tumour metastases and comprises tumours with great dependence of neovascularisation. Prostatic cancer has long been a major affliction of men, and it is becoming more common and dangerous as the population ages. It is an adenocarcinoma that is second only to lung cancer in mortality. Prostate cancer currently accounts for about 35.000 deaths each year in the United States alone. To the present time, there are no effective preventive or treatment methods for prostatic cancer. When the cancer is in its early, hormone-dependent stage, it is commonly treated by orchiectomy or chemical castration. In the later stages of prostatic cancer, the disease becomes hormone-independent and metastasises widely, usually first into the skeleton. Treatment for advanced disease initially involves hormonal manipulations and palliative radiotherapy or treatment with cytostatic agents. These strategies have proven to be of marked clinical benefit in terms of symptomatic relief but there are no effective methods, which can put prostatic cancer into remission in that stage. The use of cytotoxic agents in the management of hormone-resistant advanced prostate cancer remains poorly defined. A few single agents have become "standard therapy", although demonstration of their efficacy, by contemporary standards, is lacking. Thus, prostatic cancer is not only relatively common, but is refractory to treatment once the disease crosses into the hormone-independent stage.

In US Patent No. 4,547,511 and in EP 59,698 some derivatives of N-aryl-1,2-dihydro-4-amino-1-alkyl-2-oxo-quinoline-3-carboxamide and N-aryl-1,2-dihydro-4-hydroxy-1-alkyl-2-oxo-quinoline-3-carboxamide are claimed as enhancers of cell-mediated immunity. Roquinimex (Merck Index 12$^{th}$ Ed., No. 8418; Linomide®, N-methyl-N-phenyl-1,2-dihydro-4-hydroxy-1-methyl-2-oxo-quinoline-3-carboxamide) has been reported to have antitumour effects on Dunning R-3327 rat prostatic cancers (1).

In WO 92/18483 quinoline-3-carboxamide derivatives substituted in the 6-position with a $R_AS(O)_n$-group ($R_A$ = lower alkyl or aryl; n = 0 - 2), which possess an immunomodulating, anti-inflammatory and anti-cancer effect, are claimed. Further, WO 95/24395 and GB 2290786 disclose quinoline-3-thiocarboxamide derivatives, which possess an immunomodulating, anti-inflammatory and anti-cancer effect.

Some 5-substituted N-aryl-1,2-dihydro-4-hydroxy-1-alkyl-2-oxo-quinoline-3-carboxamides effective in the treatment of diseases resulting from autoimmunity and pathologic inflammation are known from WO-A-9955678, WO-A-0003991 and WO-A-0003992.

Roquinimex, a compound with shown anti-angiogenic and pro NK lymphocyte activities also induce pro-inflammatory side effects in humans that was emphasised when treating patients with renal cell carcinoma (2). Roquinimex in this study was poorly tolerated, with 40 % of the patients being withdrawn or having dose reductions due to adverse events, mostly influenza-like symptoms of myalgia, arthralgia and fatigue. Several cases of pericarditis and neuropathy were also observed.

Pro-inflammatory effects induced by roquinimex are efficiently monitored in the beagle dog. Roquinimex induces the beagle pain syndrome characterised by fever, myalgia, arthralgia as well as arteritis (3, 4).

DESCRIPTION OF THE INVENTION

A primary objective of the present invention is the use of quinoline derivatives for the manufacturing of a medicament for the treatment of a plurality of malignant tumours, especially in solid form. Another objective is to provide structurally novel quinoline derivatives, having a pharmacological profile that is distinguished by high potency in experimental models and low level of side effects. The types of cancer that are especially inhibited by these quinoline derivatives include, for example, breast cancers, colon cancers, Kaposi's sarcoma, lung cancers, ovarian cancers, prostatic cancers, and skin cancers. More particularly, the present invention relates to quinoline derivatives suitable for the prevention of the development of malignant tumours, in particular prostatic cancers, and prevention and treatment of the metastases of malignant tumours, particularly of prostatic cancers. To increase the cure rate of metastatic malignant tumours, in particular of prostatic cancers, an effective therapy for malignant tumour cells, particularly androgen-independent prostatic cancer cells, is needed. The approach we have chosen is to inhibit the tumour-induced angiogenesis and to stimulate the host immune system to evoke/enhance an antitumour response. In the present invention, the ability of quinoline compounds to elicit an antitumour effect against an androgen-independent Dunning R-3327 AT-1 rat prostatic cancer was tested.

It has now surprisingly been found that the compounds of general formula (1)

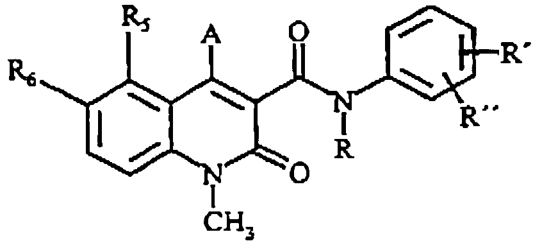

(I)

wherein

A is selected from $OR_{41}$ and $NR_{42}$ $R_{43}$ wherein

$R_{41}$ is selected from hydrogen, the pharmaceutically acceptable inorganic cations, sodium, potassium and calcium, the organic cations monoethanolamine, diethanolamine, dimethylaminoethanol, and morpholine; and $COR_A$ wherein

$R_A$ is selected from methyl, ethyl, *n*-propyl, *iso*-propyl, *tert*-butyl, *neo*-pentyl, phenyl, benzyl, and phenethyl;

$R_{42}$ and $R_{43}$ are the same or different and selected from hydrogen, methyl, ethyl, *n*-propyl, *iso*-propyl, and cyclopropyl; or

$R_{42}$ is $COR_B$ wherein

$R_B$ is C1-C4-alkyl, or

$COR_B$ is a 2-acyloxymethylbenzoyl group

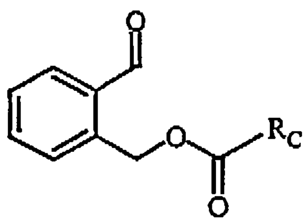

wherein $R_C$ is selected from methyl, ethyl, phenyl and benzyl;

R is selected from hydrogen, methyl, ethyl, *n*-propyl, *iso*-propyl, *n*-butyl, *iso*-butyl, *sec.*-butyl and allyl; with the proviso that R is not hydrogen when A is $OR_{41}$;

R' is selected from hydrogen, methyl, methoxy, fluoro, chloro, bromo, cyano, trifluoromethyl, and $OCH_xF_y$, wherein

$$x = 0 - 2,$$

$$y = 1 - 3$$

with the proviso that

$$x + y = 3;$$

and with the proviso that R’ is not hydrogen when R is methyl and A is $OR_{41}$;
R" is selected from hydrogen, fluoro and chloro, with the proviso that R" is selected from fluoro and chloro only when R’ is selected from fluoro and chloro;
$R_5$ is selected from hydrogen, methyl, ethyl, *n*-propyl, *iso*-propyl, methoxy, ethoxy, fluoro, chloro, bromo, trifluoromethyl, dimethylamino and $OCH_xF_y$, and $OCH_2CH_xF_y$
wherein

$$x = 0 - 2,$$

$$y = 1 - 3$$

with the proviso that

$$x + y = 3$$

and
with the further provisos that $R_5$ is not fluoro or amino when A is $OR_{41}$; and that $R_5$ is hydrogen only when A is $NR_{42}$ $R_{43}$ and R’ is trifluoromethyl;
$R_6$ is hydrogen; or
$R_5$ and $R_6$ taken together are methylenedioxy;
are unexpectedly effective and specific in the treatment of individuals suffering from advanced cancer.
The compounds of general formula (I) may exist in different tautomeric forms and all such forms where such forms exist are included herein.

In a preferred embodiment of the invention $R_5$ is selected from methyl, ethyl, methoxy, ethoxy, chloro, and bromo, or $R_5$ and $R_6$ taken together are methylenedioxy, A is selected from $OR_{41}$ and $NR_{42}$ $R_{43}$, wherein $R_{41}$ is selected from hydrogen and sodium, and wherein $R_{42}$ and $R_{43}$ are the same or different and selected from hydrogen, methyl and ethyl, R is selected from hydrogen, methyl, ethyl and *n*-propyl, especially methyl and ethyl, and R’ is selected from hydrogen, *para*-methyl, -methoxy, -chloro, and -trifluoromethyl, especially from methoxy, chloro, and trifluoromethyl when R" is hydrogen, and R" is selected from *meta’*- and *para*-fluoro provided that R’ is *ortho*-fluoro.

The invention also discloses novel compounds of the general formula (I’)

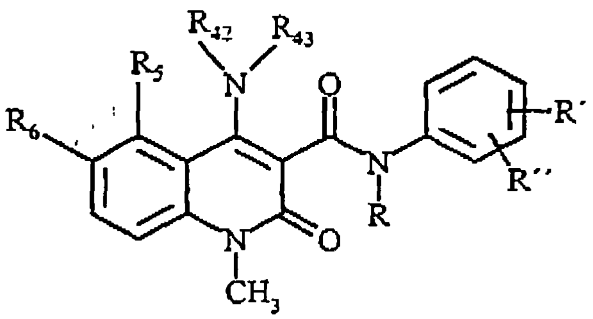

(I’)

wherein

$R_{42}$ and $R_{43}$ are the same or different and selected from hydrogen, methyl, ethyl, *n*-propyl, *iso*-propyl, and cyclopropyl; or
$R_{42}$ is $COR_B$ wherein
$R_B$ is C1-C4-alkyl, or
$COR_B$ is a 2-acyloxymethylbenzoyl group

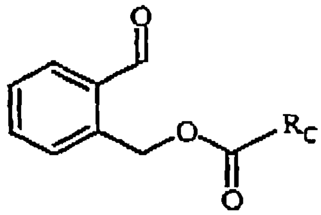

wherein $R_C$ is selected from methyl, ethyl, phenyl and benzyl;
R is selected from hydrogen, methyl, ethyl, *n*-propyl, *iso*-propyl, *n*-butyl, *iso*-butyl, *sec.*-butyl and allyl;
R' is selected from hydrogen, methyl, methoxy, fluoro, chloro, bromo, cyano, trifluoromethyl, and $OCH_xF_y$, wherein

$$x = 0 - 2,$$

$$y = 1 - 3$$

with the proviso that

$$x + y = 3;$$

R" is selected from hydrogen, fluoro and chloro, with the proviso that R" is selected from fluoro and chloro only when R' is selected from fluoro and chloro;
$R_5$ is selected from hydrogen, methyl, ethyl, *n*-propyl *iso*-propyl, methoxy, ethoxy, fluoro, chloro, bromo, trifluoromethyl, dimethylamino and $OCH_xF_y$, and $OCH_2CH_xF_y$
wherein

$$x = 0 - 2,$$

$$y = 1 - 3$$

with the proviso that

$$x + y = 3$$

and
with the proviso that $R_5$ is hydrogen only when R' is trifluoromethyl;
$R_6$ is hydrogen; or
$R_5$ and $R_6$ taken together are methylenedioxy;
or any tautomer thereof.

Among the most preferred compounds for use according to the invention are: N-ethyl-N-phenyl-1,2-dihydro-4-hydroxy-1,5-dimethyl-2-oxo-quinoline-3-carboxamide, N-etbyl-N-phenyl-1,2-dihydro-4-hydroxy-5-ethyl-1-methyl-2-oxo-quinoline-3-carboxamide, N-ethyl-N-phenyl-1,2-dihydro-4-hydroxy-5-methoxy-1-methyl-2-oxo-quinoline-3-carboxamide, N-ethyl-N-phenyl-1,2-dihydro-4-hydroxy-5-chloro-1-methyl-2-oxo-quinoline-3-carboxamide or its sodium salt, N-ethyl-N-phenyl-1,2-dihydro-4-hydroxy-5-bromo-1-methyl-2-oxo-quinoline-3-carboxamide, N-ethyl-N-phenyl-1,2-dihydro-4-hydroxy-5,6-methylenedioxy-1-methyl-2-oxo-quinoline-3-carboxamide, N-ethyl-N-phenyl-1,2-dihydro-4-hydroxy-5-dimethylamino-1-methyl-2-oxo-quinoline-3-carboxamide, N-ethyl-N-(3-fluoro-phenyl)-1,2-dihydro-4-hydroxy-5-chloro-1-methyl-2-oxo-quinoline-3-carboxamide, N-methyl-N-(4-chloro-phenyl)-1,2-dihydro-4-hydroxy-5-chloro-1-methyl-2-oxo-quinoline-3-carboxamide, N-methyl-N-(4-methyl-phenyl)-1,2-dihydro-4-hydroxy-5-chloro-1-methyl-2-oxo-quinoline-3-carboxamide, N-methyl-N-(2,4-difluoro-phenyl)-1,2-dihydro-4-hydroxy-5-chloro-1-methyl-2-oxo-quinoline-3-carboxamide, N-methyl-N-(2,5-difluoro-phenyl)-1,2-dihydro-4-hydroxy-5-chloro-1-methyl-2-oxo-quinoline-3-carboxamide, N-ethyl-N-(3-methoxy-phenyl)-1,2-dihydro-4-hydroxy-5-ethyl-1-methyl-2-oxo-quinoline-3-carboxamide, N-methyl-N-(2,4-difluorophenyl)-1,2-dihydro-4-hydroxy-5-methoxy-1-methyl-2-oxo-quinoline-3-carboxamide, N-methyl-N-(2,5-difluoro-phenyl)-1,2-dihydro-4-hydroxy-5-methoxy-1-methyl-2-oxo-quinoline-3-carboxamide, N-methyl-N-(4-trifluoromethyl-phenyl)-1,2-dihydro-4-hydroxy-5-methoxy-1-methyl-2-oxo-quinoline-3-carboxamide, N-methyl-N-(2,4-difluoro-phenyl)-1,2-dihydro-4-hydroxy-5,6-methylenedioxy-1-methyl-2-oxo-quinoline-3-carboxamide, N-methyl-N-(4-chloro-phenyl)-1,2-dihydro-4-hydroxy-1,5-dimethyl-2-oxo-quinoline-3-carboxamide, N-methyl-N-(2,4-difluoro-phenyl)-1,2-dihydro-4-hydroxy-5-ethyl-1-methyl-2-oxo-quinoline-3-carboxamide, N-methtyl-N-(4-methoxy-phenyl)-1,2-dihydro-3-dimethylamino-4-hydroxy-1-methyl-2-oxo-quinoline-3-carboxamide, N-methyl-N-phenyl-4-amino-1,2-dihydro-1,5-dimethyl-2-oxo-quinoline-3-carboxamide, N-methyl-N-phenyl-4-amino-1,2-dihydro-5-methoxy-1-methyl-2-oxo-quinoline-3-carboxamide, N-methyl-N-phenyl-5-chloro-1,2-dihydro-4-(N-methylamino)-1-methyl-2-oxo-quinoline-3-carboxamide, N-methyl-N-phenyl-1,2-dihydro-1,5-dimethyl-4-(N-methylamino)-2-oxo-quinoline-3-carboxamide, N-methyl-N-phenyl-1,2-dihydro-5-methoxy-4-(N-methylamino)-1-methyl-2-oxo-quinoline-3-carboxamide, N-methyl-N-(4-trifluoromethyl-phenyl)-4-amino-1,2-dihydro-1-methyl-2-oxo-quinoline-3-carboxamide.

High crystal lattice energy of solid compounds results in poor solubility, e.g., in water. Hence, an approach to reduce this energy will result in improved aqueous solubility. The prodrug per se is an inactive species. A basal requisite for a prodrug approach is the reconversion of the prodrug to the parent drug in vivo. The prodrug - drug conversion may take place before, during or after absorption. The necessary conversion of prodrugs to the parent drug molecules in the body can take place by a variety of reactions. The most common prodrugs are those requiring a hydrolytic cleavage mediated by enzymatic hydrolysis. In other cases, drug molecules are regenerated from their prodrugs by biochemical reductive or oxidative processes.

Esters of drugs containing a hydroxyl function have been considered as prodrug types primarily from the fact that the organism is rich in enzymes capable of hydrolysing esters. Compounds of the present invention contain an enolised carbonyl group as a prominent functional group. Under proper conditions, the enol form can be trapped by acylation of the enol group. Steric and electronic effects within the acyl group have a substantial influence upon both the aqueous and enzymatic rates of hydrolysis. Besides, physicochemical properties such as water-solubility, lipophilicity and dissolution rate can be modified for the parent compound.

Compounds of the present invention contain a 4-amino function. N-Acylation of amines to give activated amides may be a promising means of obtaining prodrug forms. 2-Hydroxy-methylbenzamides undergo a cyclisation (lactonisation) in aqueous solution to give phtalide and free amine. Substitution of the two methylene hydrogen atoms of 2-hydroxymethylbenzamide with, for example, methyl and phenyl groups greatly affects the lactonisation rates. Also blocking the lactonisation by acylation of the 2-hydroxymethyl-benzamides to give 2-acyloxymethylbenzamides affects the lactonization that must be preceded by hydrolysis of the ester grouping. Furthermore, the acylation allows control of the lipophilicity/hydrophilicity of the prodrug by the appropriate selection of the acyl group. The use of various carbamate derivatives as well as N-Mannich bases can also be considered as means to forming prodrugs.

The compounds of general formula (I) were assayed for their capacity to inhibit of growth of the Dunning R-3327 AT-1 tumour, a prostatic rat cancer. Roquinimex was used as positive treatment control and showed a 30 % inhibition at 4 mg/kg.

The compounds of general formula (I) wherein A is $NR_{42}$ $R_{43}$ are prepared by the following method:

*Method A.*

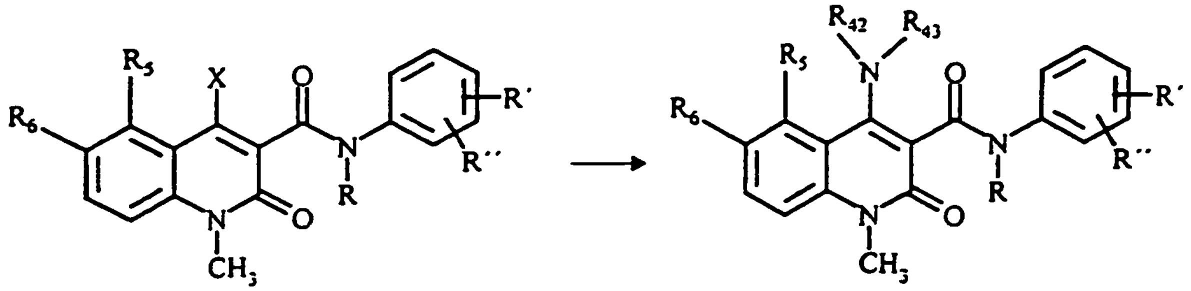

II I'

The compounds of formula (I') may be prepared by known methods, for example, by reaction of 4-chloro-1,2-dihydro-2-oxo-quinoline-3-carboxamide derivative (X = Cl; II) known from US patent No. 4,547,511 with an amino compound in a suitable alcohol, e.g., ethanol. All compounds had satisfactory $^1$H-NMR and mass spectra, although the NMR spectra were complicated due to presence of E and Z amide isomers. Only the major isomer is described by NMR-shifts below.

*Method B.*

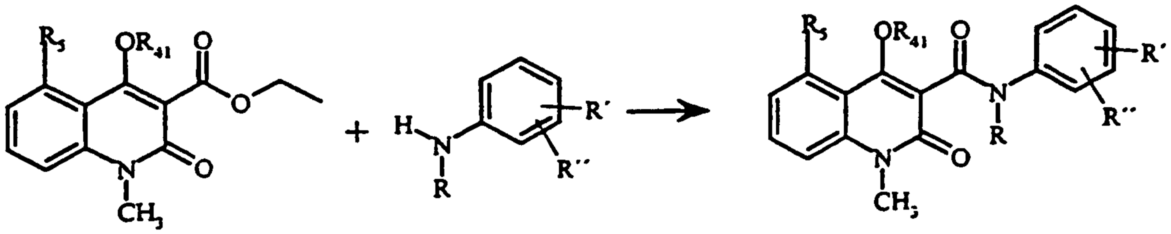

III IV I''

The compounds of general formula (I'') may be prepared by methods known from US patent No. 6,077, 851 and, for example, as shown above, by reaction of an ester derivative of the quinoline carboxylic acid with an aniline in toluene or xylene. General methods for preparation of the quinoline carboxylic acid ester derivatives of formula (III) are known from US patent No. 4,547,511. N-alkylated anilines of formula (IV) are commercially available or known from literature, e.g., in Johnstone et al, J. Chem. Soc. 1969,2223-2224. Compounds falling within the scope of formula (IV) may be prepared by methods, which are generally analogous to those of said literature.

The scope of the invention is as defined in the claims, which hereby are included by reference.

The following examples are intended to illustrate the invention without restricting the scope thereof.

*Example 1. Intermediates*

N-Methyl-N-phenyl-4-chloro-1,2-dihydro-1,5-dimethyl-2-oxo-quinoline-3-carboxamide.

N-methyl-N-phenyl-1,2-dihydro-4-hydroxy-1,5-dimethyl-2-oxo-quinoline-3-carboxamide (3.5 g) was heated in phosphorus oxytrichloride (7 ml) at 100°C for 2 hours. The mixture was concentrated, dissolved in dichloromethane and washed with water. The organic phase was dried ($Na_2SO_4$) and concentrated. The residue was triturated with ethyl acetate and filtered to give the title compound (2.3 g).

$^1$H NMR ($CDCl_3$): δ 7.50-7.00 (8H, aromatic protons), 3.60 (N-Me, s), 3.48 (N-Me, s), 2.80 (PhMe, s).

Mass (ESI, m/z) $[M+H]^+$: 341.

In essentially the same manner the following compounds were obtained from the corresponding starting materials:

N-methyl-N-phenyl-4,5-dichloro-1,2-dihydro-1-methyl-2-oxo-quinoline-3-carboxamide $^1$H NMR ($CDCl_3$ + TFA): δ 7.55-7.15 (8H, aromatic protons), 3.60 (N-Me, s), 3.47 (N-Me, s). Mass (ESI, m/z) $[M+H]^+$: 361.

N-methyl-N-(4-trifluoromethyl-phenyl)-4-chloro-1,2-dihydro-1-methyl-2-oxo-quinoline-3-carboxamide
$^1$H NMR ($CDCl_3$): δ 8.10-7.22 (8H, aromatic protons), 3.60 (N-Me, s), 3.47 (N-Me, s).
Mass (ESI, m/z) $[M+H]^+$: 395.

*Example 2.*

N-methyl-N-phenyl-1,2-dihydro-4-(N,N-dimethylamino)-1,5-dimethyl-2-oxo-quinoline-3-carboxamide.

N-Methyl-N-phenyl-4-chloro-1,2-dihydro-1,5-dimethyl-2-oxo-quinoline-3-carboxamide (0.52 g) in a mixture of dimethylamine and ethanol was heated in a thick-walled glass flask stoppered by a screw cap. The reaction was monitored by TLC (mixtures of dichloromethane/methanol were used as elutents). After 8 hours of heating the flask was cooled, opened, and diluted with water. The resulting precipitate was filtered, washed with water and dried in vacuum to afford the title compound (0.39 g).
$^1$H NMR ($CDCl_3$ + TFA): δ 7.50-6.70 (8H, aromatic protons), 3.70 (N-Me, s), 3.58 (N-Me, s), 3.50 (N-Me, s), 3.43 (N-Me, s).
Mass (ESI. m/z) $[M+H]^+$: found 350. expected 350.

In essentially the same manner the following compounds were obtained from the corresponding starting materials:

N-methyl-N-phenyl-1,2-dihydro-1,5-dimethyl-4-(N-methylamino)-2-oxo-quinoline-3-carboxamide
$^1$H NMR ($CDCl_3$ + TFA): δ 7.80-6.80 (8H, aromatic protons), 3.82 (N-Me, s), 3.59 (N-Me, s), 3.10 (N-Me, s), 1.85 (PhMe, s).
Mass (ESI, m/z) $[M+H]^+$: 336
N-methyl-N-phenyl-4-amino-1,2-dihydro-1,5-dimethyl-2-oxo-quinoline-3-carboxamide
$^1$H NMR ($CDCl_3$ + TFA): δ 7.80-6.40 (8H, aromatic protons), 3.80 (N-Me, s), 3.57 (N-Me, s). 2.28 (PhMe, s).
Mass (ESI, m/z) $[M+H]^+$: 322
N-methyl-N-phenyl-5-chloro-1,2-dihydro-4-(N,N-dimethylamino)-1-methyl-2-oxo-quinoline-3-carboxamide
$^1$H NMR ($CDCl_3$ + TFA): δ 7.70-7.00 (8H, aromatic protons), 3.68 (N-Me, s), 3.50 (N-Me, s), 3.18 ($N-Me_2$, s).
Mass (ESI, m/z) $[M+H]^+$: 370.
N-methyl-N-phenyl-5-chloro-1,2-dihydro-4-(N-methylamino)-1-methyl-2-oxo-quinoline-3-carboxamide
$^1$H NMR ($CDCl_3$ + TFA): δ 7.60-7.00 (8H, aromatic protons), 3.73 (N-Me, s), 3.57 (N-Me, s), 3.15 (N-Me, s).
Mass (ESI, m/z) $[M+H]^+$: 356.
N-methyl-N-phenyl-4-amino-5-chloro-1,2-dihydro-1-methyl-2-oxo-quinoline-3-carboxamide
$^1$H NMR ($CDCl_3$+ TFA): δ 7.62-7.23 (8H, aromatic protons), 3.63 (N-Me, s), 3.50 (N-Me, s).
Mass (ESI, m/z) $[M+H]^+$: 342.
N-ethyl-N-phenyl-5-bromo-1,2-dihydro-4-(N-ethylamino)-1-methyl-2-oxo-quinoline-3-carboxamide,
N-ethyl-N-phenyl-5-chloro-1,2-dihydro-4-(N-methylamino)-1-methyl-2-oxo-quinoline-3-carboxamide,
N-ethyl-N-phenyl-1,2-dihydro-5-ethyl-4-(N-ethylamino)-1-methyl-2-oxo-quinoline-3-carboxamide
N-ethyl-N-phenyl-1,2-dihydro-4-(N,N-dimethylamino)-5-methoxy-1-methyl-2-oxo-quinoline-3-carboxamide,
N-ethyl-N-phenyl-4-(N,N-diethylanxino)-1,2-dihydro-1-methyl-5-trifluoromethyl-2-oxo-quinoline-3-carboxamide,
N-methyl-N-phenyl-1,2-dihydxo-1,5-dimethyl-4-(N-ethylamino)-2-oxo-quinoline-3-carboxamide,
N-methyl-N-phenyl-1,2-dihydro-4-(N,N-dimethylamino)-5-methoxy-l-methyl-2-oxo-quinoline-3-carboxamide,
N-methyl-N-phenyl-1,2-dihydro-5-methoxy-1-methyl-4-(N-methylamino)-2-oxo-quinaline-3-carboxamide,
N-methyl-N-phenyl-1,2-dihydro-4-(N,N-dimethylamino)-1-methyl-5-trifluoromethyl-2-oxo-quinoline-3-carboxamide,
N-(*n*-propyl)-N-phenyl-5-chloro-1,2-dihydro-1-(N-methylamino)-1-methyl-2-oxo-quinoline-3-carboxamide,
N-*iso*-propyl-N-phenyl-1,2-dihydro-5-ethyl-4-(N-ethylamino)-1-methyl-2-oxo-quinoline-3-carboxamide.

The following two 4-amino derivatives were prepared by a slight modification of the method described above. After cooling, the reaction mixture was concentrated and dissolved in dichloromethane. The organic phase was extracted with water, dried and concentrated to furnish the product.

N-methyl-N-(4-trifluoromethyl-phenyl)-1,2-dihydro-1-methyl-4-(N-methylamino)-2-oxo-quinoline-3-carboxamide
$^1$H NMR ($CDCl_3$ + TFA): δ 7.82-7.24 (8H, aromatic protons), 3.85 (N-Me, s), 3.52 (N-Me, s), 3.18 (N-Me, s).
Mass (ESI, m/z) $[M+H]^+$ : 390.

N-methyl-N-(4-trifluoromethyl-phenyl)-1,2-dihydro-4-(N,N-dimethylamino)-1-methyl-2-oxo-quinoline-3-carboxam-

ide
$^1H$ NMR ($CDCl_3$ + TFA): δ 7.80-6.87 (8H, aromatic protons), 3.78 (N-Me, s), 3.58 (N-Me, s), 325 (N-$Me_2$, s).
Mass (ESI, m/z) $[M+H]^+$: 404.

*Example 3.*

1,2-Dihydro-4-hydroxy-5-dimethylamino-1-methyl-2-oxo-quinolline-3-carboxylic acid ethyl ester.

A solution of 2,b-difluorobensonitrile (35.7 g, 0.26 mol) and dimethylamine (17.5 g, 0.39 mol) in 100 ml of anhydrous isopropanol was heated at 110°C for 18 hours in an autoclave. After cooling, the solvent was evaporated and the residue worked up with water and diethyl ether to give a yellowish oil of 2-dimethylamino-6-fluorobensonitrile (41 g) contaminated with 2-4 % of 2,6-di(dimethylamino)bensonitrile. This crude mixture was solved in 40 % aqueous methylamine (130 ml, 1.5 mol) and ethanol (100 ml) and heated at 110°C for 18 hours in an autoclave. The product was worked up as above to give 44 g of 2-dimethylamino-6-methylaminobensonitrile (>95 % pure). This was hydrolysed in conc. sulphuric acid (170 ml) and water (34 ml) at 120°C for 3 hours. The brown solution was cooled and neutralised with 5 M NaOH. The resulting cloudy mixture was filtered through celite and washed with 30 ml of diethyl ether. The aqueous solution was extracted with dichloromethane (3x50 ml), the extracts were washed with water and evaporated to give 6-dimethylamino-N-methyl-anthranilic acid. The anthranilic acid (21.5 g, 0.11 mol) was dissolved in 250 ml of 1,4-dioxane. Phosgene (25 ml, 0.45 mol) was slowly added under cooling in an ice bath. The mixture was warmed at 40°C for 1 hour, cooled to 15°C, and the product was collected by filtration. This was worked up with aqueous sodium bicarbonate and dichloromethane, the organ phase carefully dried and evaporated to give pure 5-dimethylamino-N-methyl-isatoic anhydride. The anhydride (22 g, 0.10 mol) was dissolved in anhydrous methanol (150 ml) and sodium methoxide (5.4 g, 0.10 mol) was added. After stirring at 50°C for 3 hours, the solvent was removed and the residue worked up with water and ether to give a yellow oil (15 g). The oil, 6-dimethylamino-N-methyl-anthranilic acid methyl ester (10.4 g, 0.05 mol), was dissolved in dichloromethane (100 ml) and cooled on an ice-bath. Ethyl malonyl chloride (10 g, 0.07 mol) was added. After being stirred for 1 hour at room temperature the cloudy mixture was washed with aqueous sodium bicarbonate. The organic phase was carefully dried and concentrated under vacuum. The residue was dissolved in dry ethanol (100 ml) and sodium methoxide (9 g, 0.16 mol) was added. The mixture was stirred for 1 hour and neutralised with hydrochloric acid. The solvent was removed and the residue worked up with water and dichloromethane. The organic phase was dried and the solvent removed to give the title compound as pure, greyish crystals (11 g).
$^1H$ NMR (CDCl3) δ 1.36 (3H, t), 2.78 (6H, s), 3.59 (3H, s), 4.39 (2H, q), 7.17 (1H, d), 7.21 (1H, d), 7.54 (1H, t), 17.1 (1H, s).

*Example 4.*

N-Ethyl-N-phenyl-1,2-dihydro-5-dimethylamino-4-hydroxy-1-methyl-2-oxo-quinoline-3-carboxamide (Method B).

N-Ethylaniline (4.4 g, 0.036 mol) was dissolved in 80 ml of toluene. About 30 ml of the solvent was distilled off in order to obtain a dry solution. 1,2-Dihydro-4-hydroxy-5-dimethylamino-1-methyl-2-oxo-quinoline-3-carboxylic acid ethyl ester (3.5 g, 0.012 mol) was added to the boiling solution. The ethanol formed during the reaction was distilled off together with some toluene for about 10 hours. The mixture was cooled to room temperature.
The precipitate was collected, washed with cold toluene and hexane and dried to give the title compound (3.1 g), yield 71 %.
$^1H$ NMR (CDCl3) δ 1.25 (3H, t), 2.63 (3H, s), 2.78 (3H, s), 3.51 (3H, s), 3.87-4.07 (2H, m), 7.07-7.19 (5H, m), 7.39-7.48 (3H, m).
$^{13}C$ NMR (CDCl3) δ 13.2 (CH3), 29.4 (CH3), 43.7 (CH2), 45.2 (CH3), 46.5 (CH3), 109.6 (C), 110.4 (C), 113.0 (CH), 114.0 (CH), 127.3 (CH), 127.5 + 137.5 (CH), 128.3 + 128.3 (CH), 130.8 (CH), 140.7 (C), 142.3 (C), 150.6 (C), 159.7 (C), 160.6 (C=O), 165.0 (C=O).
Mass (ESI m/z) $[M+H]^+$ 366, fragment 245.
(25 ml), dried and evaporated to dryness to give the title compound as 1.3 g syrup.
$^1H$ NMR (CDCl3) δ 2.88 (3H, s), 6.70 (2H, d), 7.27 (1H, t), 7.41 (2H, t), 7.48 (2H, d), 7.57 (2H, d).
All embodiments of the invention as disclosed in the claims are herewith included in the specification.

*Pharmacological methods*

*Primary screen*

R-3327 AT-1 Prostatic cancer in man is an angiogenic type of solid tumour. The Dunning R-3327 AT-1 is a prostatic cancer of the rat and suits as an experimental animal model for this disease. The AT-1 tumour is serially

transplanted subcutaneously (sc) on syngeneic rats of the Copenhagen strain. Small pieces of the tumour are transplanted sc to recipient rats and treatment of the tumour bearing rats start when the tumours are easily measurable approximately on day 10 after transplantation. Doses of the compounds are given either orally or parentally 5 days a week for four weeks. The tumour growth and body weight gain are monitored during the experimental time.

*Assays for evaluation of pro-inflammatory activity*

Beagle Pain Syndrome

The Beagle Pain Syndrome (BPS) is reflected by clinical and laboratory manifestations, e.g., fever, increased erythrocyte sedimentation rate (ESR), alkaline phosphate (AP), induction of acute phase proteins and vasculitis, justifying BPS as a model for the flu-like syndrome induced by roquinimex in man. BPS is a disease of acute inflammatory nature. Several alterations in hematology and clinical chemistry are occurring. The compounds were administrated intravenously to beagle dogs. The dosage was given for five consecutive days. The dogs were evaluated for a pro-inflammatory reaction, i.e., increased ESR and AP. The percentage change of the laboratory values between Day 8 and baseline was determined.

Neutrophil inflammation

Neutrophil inflammation (NI) is induced by intradermal injection of carrageenin or zymosan. Accumulation of neutrophils is evaluated by measuring the neutrophil enzyme myeloperoxidase activity from challenged skin.

Oedema formation

Oedema formation is induced by intradermal injection of carrageenin. The extent of oedema is evaluated by measuring the plasma extravasation of intravenously injected Evans blue in challenged skin.

Delayed Type Hypersensitivity

Delayed Type Hypersensitivity (DTH) reactions are T-cell mediated and antigen-specific inflammatory reactions useful for studies on modulation of immunological and inflammatory events. Mice are sensitised to oxazolone by cutaneous administration. Challenge is performed topically on the ears and the inflammatory reaction is quantified by measuring change in ear thickness.

The following examples are intended to illustrate the invention without restricting the scope thereof.

Among preferred compounds are N-ethyl-N-phenyl-1,2-dihydro-4-hydroxy-5-chloro-1-methyl-2-oxo-quinoline-3-carboxamide, N-methyl-N-(4-chloro-phenyl)-1,2-dihydro-4-hydroxy-5-chloro-1-methyl-2-oxo-quinoline-3-carboxamide, and N-methyl-N-phenyl-4-amino-1,2-dihydro-1,5-dimethyl-2-oxo-quinoline-3-carboxamide hereinafter-called Compound A, B, and D, respectively. Roquinimex is included as a reference compound hereinafter called Compound E. In addition, the antineoplastic agents vinblastine (Merck Index 12$^{th}$ Ed., No. 10119) and doxorubicin (Merck Index 12$^{th}$ Ed., No, 3495) have been included as reference compounds.

*Tumour growth inhibition*

| Compound | Dose (mg/kg) | % Tumour Weight Reduction |
|---|---|---|
| A (invention) | 2(sc) | 37 |
| B (invention) | 2 (po) | 28 |
| D (invention) | 2 (sc) | 38 |
| E (reference) | 4 (sc) | 30 |
| vinblastine (reference) | 1 (iv)[a)] | 0 |
| vinblastine (reference) | 4 (iv) [a)] | toxic |
| doxorubicin (reference) | 4 (ip) [a)] | 0 |

(continued)

| Compound | Dose (mg/kg) | % Tumour Weight Reduction |
|---|---|---|
| doxorubicin (reference) | 8 (ip) [a)] | 31 [b)] |
| [a)] treatment given on Day 11<br>[b)] 8 mg/kg of doxorubicin caused a body weight loss | | |

*Beagle Pain Syndrome - percentage change (Day 8 - baseline)*

| Dose, mg/kg p.o. | Compound A (invention) | | Compound E (reference) | |
|---|---|---|---|---|
| | ESR | AP | ESR | AP |
| 1 | 177 | 25 | 700 | 103 |
| 10 | 515 | 109 | | |

The administration of compounds of formula I in order to practice the present methods of therapy is carried out by administering an effective amount of the chosen compound to the patient in need of such treatment or prophylaxis. The effective amount of an individual compound is determined by a physician and depends on factors such as the exact disease to be treated, the severity of the disease and other diseases or conditions from which the patient suffers, and other factors in tile physician's judgement. It will be observed that the compounds are active at low dosage levels, thereby allowing effective treatment or prophylaxes with slight probability of side effects or cross-reactions with other treatments or drugs. A suitable daily dose for use in the treatment of the disease is contemplated to vary between 0.002 mg/kg to about 100 mg/kg body weight, in particular between 0.02 mg/kg to 10 mg/kg body weight, depending upon the specific condition, e.g., prostatic cancer, to be treated, the age and weight of the specific patient, and the specific patient's response to the medication. The exact individual dosage, as well as the daily dosage, will be determined according to standard medical principles under the direction of a physician.

Effective quantities of the compounds of formula (I) are preferably administered to a patient in need of such treatment according to usual routes of administration and formulated in usual pharmaceutical compositions comprising an effective amount of the active ingredient and a suitable pharmaceutically acceptable carrier. By "pharmaceutically acceptable" it is meant the carrier, diluent or excipient must be compatible with the other ingredients of the formulation and not deleterious to the recipient thereof. Such compositions may take a variety of forms, e.g. solutions, suspensions, emulsions, tablets, capsules, and powders prepared for oral administration, sterile solutions for parental administration, suppositories for rectal administration or suitable topical formulations. In making the composition of the present invention, the active ingredient will usually be admixed with a carrier, or diluted by a carrier, or enclosed within a carrier that may be in the form of a capsule, sachet, paper, or other container. When the carrier serves as a diluent, it may be a solid, semi-solid or liquid material that acts as a vehicle, excipient or medium for the active ingredient. Various additives to enhance the stability or ease of administration of the drug are contemplated. Thus, the compositions can be in the form of tablets, pills, powders, lozenges, sachets, cachets, elixirs, suspensions, emulsions solutions, syrups, aerosols (as a solid or in a liquid medium), soft and hard gelatine capsules, suppositories, sterile injectable solutions, sterile packaged powders, and the like. Conventional procedures for the selection and preparation of suitable pharmaceutical formulations are described, for example, in "Pharmaceuticals - The Science of Dosage Form Design", M.B. Aulton, Churchill Livingstone, 1988. The pharmaceutical composition may also contain additional therapeutically useful substances other than a compound of formula (I).

*References*

1. Ichikawa, T. et al., The antitumor effects of the quinoline-3-carboxamide Linomide on Dunning R-3327 rat prostatic cancers. Cancer Research 52:3022-3028, 1992.
2. de Wit, R,. et al., EORTC phase II study of daily oral linomide in metastatic renal cell carcinoma patients with good prognostic factors. Eur. J. Cancer 33:493-495, 1997.
3. Kelly, D.F., Grimsell, C.S.G. and Kenyon, C.J., Polyarteritis in the dog: A case report. Vet Record 92: 363-366, 1973.
4. Harcourt, R.A., Polyarterites in a colony of beagles. Vet Record 102: 519-522, 1978.

## Claims

1. Use of compounds of the general formula (I)

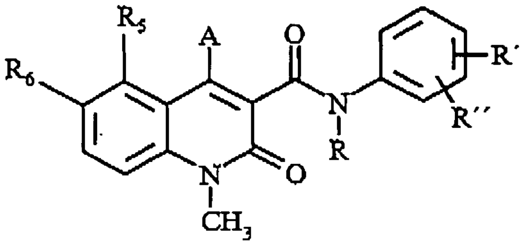

(I)

wherein
A is selected from $OR_{41}$ and $NR_{42}R_{43}$ wherein
$R_{41}$ is selected from hydrogen, the pharmaceutically acceptable inorganic cations, sodium, potassium and calcium; the organic cations monoethanolamine, diethanolamine, dimethylaminoethanol and morpholine; and $COR_A$ wherein $R_A$ is selected from methyl, ethyl, n-propyl, iso-propyl, tert-butyl, neo-pentyl, phenyl, benzyl and phenethyl;
$R_{42}$ and $R_{43}$ are the same or different and selected from hydrogen, methyl, ethyl, n-propyl, iso-propyl, and cyclopropyl; or
$R_{42}$ is $COR_B$ wherein
$R_B$ is C1-C4-alkyl, or
$COR_B$ is a 2-acyloxymethylbenzoyl group

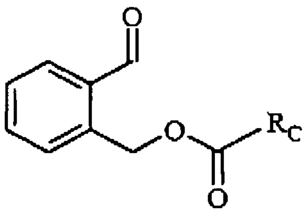

wherein $R_C$ is selected from methyl, ethyl, phenyl and benzyl;
R is selected from hydrogen, methyl, ethyl, n-propyl, iso-propyl, n-butyl, iso-butyl, sec.-butyl and allyl; with the proviso that R is not hydrogen when A is $OR_{41}$;
R' is selected from hydrogen, methyl, methoxy, fluoro, chloro, bromo, cyano, trifluoromethyl, and $OCH_xF_y$, wherein

$$x = 0 - 2,$$

$$y = 1 - 3$$

with the proviso that

$$x + y = 3;$$

and with the proviso that R' is not hydrogen when R is methyl and A is $OR_{41}$;
R" is selected from hydrogen, fluoro and chloro, with the proviso that R" is selected from fluoro and chloro only when R' is selected from fluoro and chloro;
$R_5$ is selected from hydrogen, methyl, ethyl, n-propyl, iso-propyl, methoxy, ethoxy, fluoro, chloro, bromo, trifluoromethyl, dimethylamino and $OCH_xF_y$, and $OCH_2CH_xF_y$

wherein

$$x = 0 - 2,$$

$$y = 1 - 3$$

with the proviso that

$$x + y = 3$$

and
with the further provisos that $R_5$ is not fluoro when A is $OR_{41}$; and that $R_5$ is hydrogen only when A is $NR_{42}R_{43}$ and R' is trifluoromethyl;
$R_6$ is hydrogen; or
$R_5$ and $R_6$ taken together are methylenedioxy;
or any tautomer thereof;
for the manufacturing of a medicament for the treatment of a solid malignant tumour disease.

**2.** Use according to claim 1 wherein the solid malignant tumor disease is selected from breast cancers, colon cancers, Kaposi's sarcoma, lung cancers, ovarian cancers, prostatic cancers and skin cancers.

**3.** Use according to claim 2 wherein the solid malignant tumor disease is breast cancer or prostatic cancer.

**4.** Use according to any of the claims 1-3 of compounds wherein A is $OR_{41}$.

**5.** Use according to any of the claims 1-3 of compounds wherein A is $NR_{42}R_{43}$.

**6.** Use according to any of the claims 1-4 of compounds wherein $R_5$ is methyl, ethyl, methoxy, chloro, bromo, or $R_5$ and $R_6$ taken together are methylenedioxy.

**7.** Use according to any of the claims 1-4 and 6 of compounds wherein R is methyl and ethyl.

**8.** Use according to any of the claims 1-4 and 7 of compounds wherein R is ethyl and R' is hydrogen.

**9.** Use according to any of the claims 1-4 and 6 of compounds wherein R' is selected from para-methyl, -methoxy, -chloro, -trifluoromethyl.

**10.** Use according to any of the claims 1-4 and 6 of compounds wherein R" is selected from meta'- and para-fluoro provided that R' is ortho-fluoro.

**11.** Use according to claim 4 of compounds wherein $R_{41}$ is selected from hydrogen and sodium.

**12.** Use according to claim 4 of the compound, N-ethyl-N-phenyl-1,2-dihydro-4-hydroxy-1,5-dimethyl-2-oxo-quinoline-3-carboxamide.

**13.** Use according to claim 4 of the compound, N-ethyl-N-phenyl-1,2-dihydro-4-hydroxy-5-ethyl-1-methyl-2-oxo-quinoline-3-carboxamide.

**14.** Use according to claim 4 of the compound, N-ethyl-N-phenyl-X,2-dihydro-4-hydroxy-5-methoxy-1-methyl-2-oxo-quinoline-3-carboxamide.

**15.** Use according to claim 4 of the compound, N-ethyl-N-phenyl-1,2-dihydro-4-hydroxy-5-chloro-1-methyl-2-oxo-quinoline-3-carboxamide or its sodium salt.

**16.** Use according to claim 4 of the compound, N-ethyl-N-phenyl-1,2,dihydro-4-hydroxy-5-bromo-1-methyl-2-oxo-quinoline-3-carboxamide.

**17.** Use according to claim 4 of the compound, N-ethyl-N-phenyl-1,2-dihydro-4-hydroxy-5,6-methylenedioxy-1-methyl-2-oxo-quinoline-3-carboxamide.

**18.** Use according to claim 4 of the compound N-ethyl-N-phenyl-1,2-dihydro-4-hydroxy-5-dimethylamino-1-methyl-2-oxo-quinoline-3-carboxamide.

**19.** Use according to claim 4 of the compound, N-ethyl-N-(3-fluoro-phenyl)-1,2-dihydro-4-hydroxy-5-chlozo-1-methyl-2-oxo-quinoline-3-carboxamide.

**20.** Use according to claim 4 of the compound N-methyl-N-(4-chloro-phenyl)-1,2-dihydro-4-hydroxy-5-chloro-1-methyl-2-oxo-quinoline-3-carboxamide.

**21.** Use according to claim 4 of the compound N-methyl-N-(4-methyl-phenyl)-1,2-dihydro-4-hydroxy-5-chloro-1-methyl-2-oxo-quinoline-3-carboxamide.

**22.** Use according to claim 4 of the compound, N-methyl-N-(2,4-difluoxo-phenyl)-1,2-dihydro-4-hydroxy-5-chloro-1-methyl-2-oxo-quinoline-3-carboxamide.

**23.** Use according to claim 4 of the compound, N-methyl-N-(2,5-difluoro-phenyl)-1,2-dihydro-4-hydroxy-5-chloro-1-methyl-2-oxo-quinoline-3-carboxamide.

**24.** Use according to claim 4 of the compound, N-ethyl-N-(3-methoxy-phenyl)-1,2-dihydro-4-hydroxy-5-ethyl-1-methyl-2-oxo-quinoline-3-carboxamide.

**25.** Use according to claim 4 of the compound, N-ethyl-N-(4-chloro-phenyl)-1,2-dihydro-4-hydroxy-1,5-dimethyl-2-oxo-quinoline-3-carboxamide.

**26.** Use according to claim 4 of the compound, N-methyl-N-(2,4-difluoro-phenyl)-1,2-dihydro-4-hydroxy-5-ethyl-1-methyl-2-oxo-quinoline-3-carboxamide.

**27.** Use according to claim 4 of the compound, N-methyl-N-(2,4-difluoro-phenyl)-1,2-dihydro-4-hydroxy-5-methoxy-1-methyl-2-oxo-quinoline-3-carboxamide.

**28.** Use according to claim 4 of the compound, N-methyl-N-(2,5-difluorophenyl)-1,2-dihydro-4-hydroxy-5-methoxy-1-methyl-2-oxo-quinoline-3-carboxamide.

**29.** Use according to claim 4 of the compound, N-methyl-N-(4-trifluoronaethyl-phenyl)-1,2-dihydro-4-hydroxy-5-methoxy-1-methyl-2-oxo-quinoline-3-carboxamide.

**30.** Use according to claim 4 of the compound, N-methyl-N-(2,4-difluoro-phenyl)-1,2-dihydro-4-hydroxy-5,6-methylenedioxy-1-methyl-2-oxo-quinoline-3-carboxamide.

**31.** Use according to claim 4 of the compound, N-methyl-N-(4-methoxy-phenyl)-1,2-dihydro-5-dimethylamino-4-hydroxy-1-methyl-2-oxo-quinoline-3-carboxamide.

**32.** Use according to any of the claims 1-3 and 5 of compounds wherein $R_5$ is selected from methyl, ethyl, methoxy, ethoxy, chloro, and bromo, or $R_5$ and $R_6$ taken together are methylenedioxy.

**33.** Use according to any of the claims 1-3, 5 and 32 of compounds wherein R is selected from methyl, ethyl, n-propyl.

**34.** Use according to any of the claims 1-3, 5 and 32 of compounds wherein R is hydrogen.

**35.** Use according to any of the claims 1-3, 5 and 32 of compounds wherein R is methyl and R' is hydrogen.

**36.** Use according to any of the claims 1-3, 5 and 32 of compounds wherein $R_{42}$ and $R_{43}$ are hydrogen.

**37.** Use according to any of the claims 1-3, 5 and 32 of compounds wherein R' is selected from hydrogen, para-methyl, -methoxy, -chloro, and -trifluoromethyl.

**38.** Use according to any of the claims 1-3, 5 and 32 of compounds wherein R" is selected from meta'- and para-fluoro provided that R' is ortho-fluoro.

**39.** Use according to claim 5 of the compound N-methyl-N-phenyl-4-amino-1,2-dihydro-1,5-dimethyl-2-oxo-quinoline-3-carboxamide.

**40.** Use according to claim 5 of the compound N-methyl-N-phenyl-4-amino-1,2-dihydro-5-methoxy-1-methyl-2-oxo-quinoline-3-carboxamide.

**41.** Use according to claim 5 of the compound N-methyl-N-phenyl-1,2-dihydro-1,5-dimethyl-4-(N-methylamino)-2-oxo-quinoline-3-carboxamide.

**42.** Use according to claim 5 of the compound N-methyl-N-phenyl-1,2-dihydro-5-methoxy-4-(N-methylamino)-1-methyl-2-oxo-quinoline-3-carboxamide.

**43.** Use according claim 5 of the compound N-methyl-N-(4-trifluoromethyl)phenyl-4-amino-1,2-dihydro-1-methyl-2-oxo-quinoline-3-carboxamide.

**44.** A compound of formula (I)

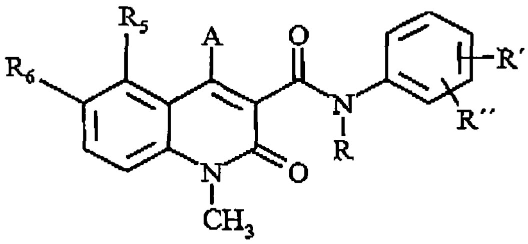

(I)

wherein
A is $NB_{42}R_{43}$ wherein
$R_{42}$ and $R_{43}$ are the same or different and selected from hydrogen, methyl, ethyl, n-propyl, iso-propyl, and cyclopropyl;
or
$R_{42}$ is $COR_B$ wherein
$R_B$ is C1-C4-alkyl, or
$COR_B$ is a 2-acyloxymethylbenzoyl group

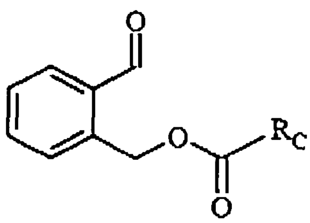

wherein $R_C$ is selected from methyl, ethyl, phenyl and benzyl;
R is selected from hydrogen, methyl, ethyl, n-propyl, iso-propyl, n-butyl, iso-butyl, sec.-butyl and allyl;
R' is selected from hydrogen, methyl, methoxy, fluoro, chloro, bromo, cyano, trifluoromethyl, and $OCH_xF_y$,
wherein

$$x = 0 - 2,$$

$$y = 1 - 3$$

with the proviso that

$$x + y = 3;$$

R" is selected from hydrogen, fluoro and chloro, with the proviso that R" is selected from fluoro and chloro only when R’ is selected from fluoro and chloro;
$R_5$ is selected from hydrogen, methyl, ethyl, n-propyl, iso-propyl, methoxy, ethoxy, fluoro, chloro, bromo, trifluoromethyl, dimethylamino and $OCH_xF_y$, and $OCH_2CH_xF_y$
wherein

$$x = 0 - 2,$$

$$y = 1 - 3$$

with the proviso that

$$x + y = 3$$

and
with the proviso that $R_5$ is hydrogen only when R’ is trifluoromethyl ;
$R_6$ is hydrogen ; or
$R_5$ and $R_6$ taken together are methylenedioxy;
or any tautomer thereof.

**45.** Compound according to claim 44 wherein $R_5$ is selected from methyl, ethyl, methoxy, ethoxy, or $R_5$ and $R_6$ taken together are methylenedioxy.

**46.** Compound according to claim 44 or 45 wherein R is selected from methyl, ethyl and n-propyl.

**47.** Compound according to claim 44 or 45 wherein R is hydrogen.

**48.** Compound according to claim 44 or 45 wherein R is methyl and R’ is hydrogen.

**49.** Compound according to claim 44 or 45 wherein $R_{42}$ and $R_{43}$ are hydrogen.

**50.** Compound according to claim 44 or 45 wherein R’ is selected from hydrogen and trifluoromethyl.

**51.** The compound according to claim 44 which is N-methyl-N-phenyl-4-amino-1,2-dihydro-1,5-dimethyl-2-oxo-quinoline-3-carboxamide.

**52.** The compound according to claim 44 which is N-methyl-N-phenyl-4-amino-1,2-dihydro-5-methoxy-1-methyl-2-oxo-quinoline-3-carboxamide.

**53.** The compound according to claim 44 which is N-methyl-N-phenyl-1,2-dihydro-1,5-dimethyl-4-(N-methylamino)-2-oxo-quinoline-3-carboxamide.

**54.** The compound according to claim 44 which is N-methyl-N-phenyl-1,2-dihydra-5-methoxy-4-(N-methylamino)-1-methyl-2-oxo-quinoline-3-carboxamide.

**55.** The compound according to claim 44 which is N-methyl-N-(4-trifluoronlethyl)phenyl-4-amino-1,2-dihydro-1-methyl-2-oxo-quinoline-3-carboxamide.

**56.** The compound N-ethyl-N-phenyl-1,2-dihydro-4-hydroxy-5-dimethylamino-1-methyl-2-oxo-quinoline-3-carboxamide.

**57.** The compound N-methyl-N-(4-methoxy-phenyl)-1,2-dihydro-5-dimethylamino-4-hydroxy-1-methyl-2-oxo-quinoline-3-carboxamide.

**58.** The compound according to any of the claims 44 to 57 to be used as therapeutic.

**59.** Pharmaceutical composition comprising as active ingredient a compound according to any of the claims 44-57 together with a pharmaceutically acceptable carrier.

**60.** Pharmaceutical compositions according to claim 59 comprising other therapeutically active substances.

**61.** Pharmaceutical compositions according to claim 59 in dosage form sufficient to provide a daily dosage of the active substance of 0.002 mg/kg to about 100 mg/kg body weight, in particular between 0.02 mg/kg to 10 mg/kg body weight.

**62.** A process for the preparation of a compound according to any of claims 44 to 55 by reaction of a 4-chloro-1,2-dihydro-2-oxo-quinoline-3-carboxamide derivative of formula (II)

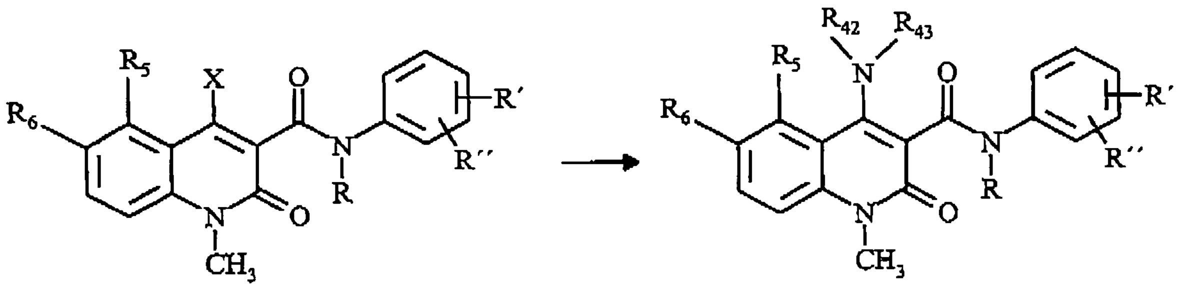

II I′

with an amino compound in a suitable alcohol, e.g., ethanol.

**Patentansprüche**

**1.** Verwendung von Verbindungen der allgemeinen Formel (I)

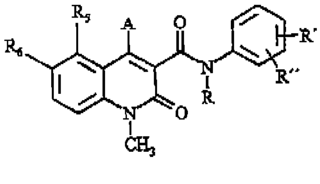

(I)

worin
A ausgewählt ist aus $OR_{41}$ und $NR_{42}R_{43}$, worin
$R_{41}$ ausgewählt ist aus Wasserstoff, den pharmazeutisch annehmbaren anorganischen Kationen, Natrium, Kalium und Calcium; den organischen Kationen Monoethanolamin, Diethanolamin, Dimethylaminoethanol und Morpholin; und $COR_A$, worin

$R_A$ ausgewählt ist aus Methyl, Ethyl, n-Propyl, Isopropyl, tert-Butyl, Neopentyl, Phenyl, Benzyl und Phenethyl;
$R_{42}$ und $R_{43}$ gleich oder voneinander verschieden sind und ausgewählt sind aus Wasserstoff, Methyl, Ethyl, n-Propyl, Isopropyl und Cyclopropyl; oder
$R_{42}$ $COR_B$ ist, worin
$R_B$ $C_1$-$C_4$-Alkyl ist, oder
$COR_B$ eine 2-Acyloxymethylbenzoylgruppe ist

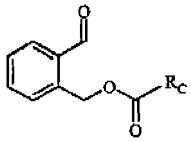

worin $R_C$ ausgewählt ist aus Methyl, Ethyl, Phenyl und Benzyl;
R ausgewählt ist aus Wasserstoff, Methyl, Ethyl, n-Propyl, Isopropyl, n-Butyl, Isobutyl, sec-Butyl und Allyl; mit der Maßgabe, dass R nicht Wasserstoff ist, wenn A $OR_{41}$ ist:
R' ausgewählt ist aus Wasserstoff, Methyl, Methoxy, Fluor, Chlor, Brom, Cyano, Trifluormethyl und $OCH_xF_y$, worin

$$x = 0 - 2,$$

$$y = 1 - 3$$

mit der Maßgabe, dass

$$x + y = 3;$$

und mit der Maßgabe, dass R' nicht Wasserstoff ist, wenn R Methyl ist und A $OR_{41}$ ist;
R" ausgewählt ist aus Wasserstoff, Fluor und Chlor, mit der Maßgabe, dass R" nur aus Fluor und Chlor ausgewählt ist, wenn R' aus Fluor und Chlor ausgewählt ist;
$R_5$ ausgewählt ist aus Wasserstoff, Methyl, Ethyl, n-Propyl, Isopropyl, Methoxy, Ethoxy, Fluor, Chlor, Brom, Trifluormethyl, Dimethylamino und $OCH_xF_y$, und $OCH_2CH_xF_y$,
worin

$$x = 0 - 2,$$

$$y = 1 - 3$$

mit der Maßgabe, dass

$$x + y = 3$$

und
mit den weiteren Maßgaben, dass $R_5$ nicht Fluor ist, wenn A $OR_{41}$ ist: und dass $R_5$ nur Wasserstoff ist, wenn A $NR_{42}R_{43}$ ist und R' Trifluormethyl ist;
$R_6$ Wasserstoff ist; oder
$R_5$ und $R_6$ zusammengenommen Methylendioxy sind;
oder einem beliebigen Tautomer davon;
zum Herstellen eines Medikaments für die Behandlung einer soliden malignen Tumorerkrankung.

2. Verwendung nach Anspruch 1, wobei die solide maligne Tumorerkrankung ausgewählt ist aus Brustkrebsen, Dickdarmkrebsen, Kaposi-Sarkom, Lungenkrebsen, Eierstockkrebsen, Prostatakrebsen und Hautkrebsen.

**3.** Verwendung nach Anspruch 2, wobei die solide maligne Tumorerkrankung Brustkrebs oder Prostatakrebs ist.

**4.** Verwendung nach einem der Ansprüche 1 - 3 von Verbindungen, worin A $OR_{41}$ ist.

**5.** Verwendung nach einem der Ansprüche 1 - 3 von Verbindungen, worin A $NR_{42}R_{43}$ ist.

**6.** Verwendung nach einem der Ansprüche 1 - 4 von Verbindungen, worin $R_5$ Methyl, Ethyl, Methoxy, Chlor, Brom ist oder $R_5$ und $R_6$ zusammengenommen Methylendioxy sind.

**7.** Verwendung nach einem der Ansprüche 1 - 4 und 6 von Verbindungen, worin R Methyl und Ethyl ist.

**8.** Verwendung nach einem der Ansprüche 1 - 4 und 7 von Verbindungen, worin R Ethyl ist und R' Wasserstoff ist.

**9.** Verwendung nach einem der Ansprüche 1 - 4 und 6 von Verbindungen, worin R' ausgewählt ist aus para-Methyl, -Methoxy, -Chlor, -Trifluormethyl.

**10.** Verwendung nach einem der Ansprüche 1 - 4 und 6 von Verbindungen, worin R" ausgewählt ist aus meta'- und para-Fluor, mit der Maßgabe, dass R' ortho-Fluor ist.

**11.** Verwendung nach Anspruch 4 von Verbindungen, worin $R_{41}$ ausgewählt ist aus Wasserstoff und Natrium.

**12.** Verwendung nach Anspruch 4 der Verbindung N-Ethyl-N-phenyl-1,2-dihydro-4-hydroxy-1,5-dimethyl-2-oxo-chinolin-3-carboxamid.

**13.** Verwendung nach Anspruch 4 der Verbindung N-Ethyl-N-phenyl-1,2-dihydro-4-hydroxy-5-ethyl-1-methyl-2-oxo-chinolin-3-carboxamid.

**14.** Verwendung nach Anspruch 4 der Verbindung N-Ethyl-N-phenyl-1,2-dihydro-4-hydroxy-5-methoxy-1-methyl-2-oxo-chinolin-3-carboxamid.

**15.** Verwendung nach Anspruch 4 der Verbindung N-Ethyl-N-phenyl-1,2-dihydro-4-hydroxy-5-chlor-1-methyl-2-oxo-chinolin-3-carboxamid oder ihres Natriumsalzes.

**16.** Verwendung nach Anspruch 4 der Verbindung N-Ethyl-N-phenyl-1,2-dihydro-4-hydroxy-5-brom-1-methyl-2-oxo-chinolin-3-carboxamid.

**17.** Verwendung nach Anspruch 4 der Verbindung N-Ethyl-N-phenyl-1,2-dihydro-4-hydroxy-5,6-methylendioxy-1-methyl-2-oxo-chinolin-3-carboxamid.

**18.** Verwendung nach Anspruch 4 der Verbindung N-Ethyl-N-phenyl-1,2-dihydro-4-hydroxy-5-dimethylamino-1-methyl-2-oxo-chinolin-3-carboxamid.

**19.** Verwendung nach Anspruch 4 der Verbindung N-Ethyl-N-(3-fluor-phenyl)-1,2-dihydro-4-hydroxy-5-chlor-1-methyl-2-oxo-chinolin-3-carboxamid.

**20.** Verwendung nach Anspruch 4 der Verbindung N-Methyl-N-(4-chlor-phenyl)-1,2-dihydro-4-hydroxy-5-chlor-1-methyl-2-oxo-chinolin-3-carboxamid.

**21.** Verwendung nach Anspruch 4 der Verbindung N-Methyl-N-(4-methyl-phenyl)-1,2-dihydro-4-hydroxy-5-chlor-1-methyl-2-oxo-chinolin-3-carboxamid.

**22.** Verwendung nach Anspruch 4 der Verbindung N-Methyl-N-(2,4-difluor-phenyl)-1,2-dihydro-4-hydroxy-5-chlor-1-methyl-2-oxo-chinolin-3-carboxamid.

**23.** Verwendung nach Anspruch 4 der Verbindung N-Methyl-N-(2,5-difluor-phenyl)-1,2-dihydro-4-hydroxy-5-chlor-1-methyl-2-oxo-chinolin-3-carboxamid.

**24.** Verwendung nach Anspruch 4 der Verbindung N-Ethyl-N-(3-methoxy-phenyl)-1,2-dihydro-4-hydroxy-5-ethyl-1-methyl-2-oxo-chinolin-3-carboxamid.

**25.** Verwendung nach Anspruch 4 der Verbindung N-Ethyl-N-(4-chlor-phenyl)-1,2-dihydro-4-hydroxy-1,5-dimethyl-2-oxo-chinolin-3-carboxamid.

**26.** Verwendung nach Anspruch 4 der Verbindung N-Methyl-N-(2,4-difluor-phenyl)-1,2-dihydro-4-hydroxy-5-ethyl-1-methyl-2-oxo-chinolin-3-carboxamid.

**27.** Verwendung nach Anspruch 4 der Verbindung N-Methyl-N-(2,4-difluor-phenyl)-1,2-dihydro-4-hydroxy-5-methoxy-1-methyl-2-oxo-chinolin-3-carboxamid.

**28.** Verwendung nach Anspruch 4 der Verbindung N-Methyl-N-(2,5-difluor-phenyl)-1,2-dihydro-4-hydroxy-5-methoxy-1-methyl-2-oxo-chinolin-3-carboxamid.

**29.** Verwendung nach Anspruch 4 der Verbindung N-Methyl-N-(4-trifluormethyl-phenyl)-1,2-dihydro-4-hydroxy-5-methoxy-1-methyl-2-oxo-chinolin-3-carboxamid.

**30.** Verwendung nach Anspruch 4 der Verbindung N-Methyl-N-(2,4-difluor-phenyl)-1,2-dihydro-4-hydroxy-5,6-methylendioxy-1-methyl-2-oxo-chinolin-3-carboxamid.

**31.** Verwendung nach Anspruch 4 der Verbindung N-Methyl-N-(4-methoxy-phenyl)-1,2-dihydro-5-dimethylamino-4-hydroxy-1-methyl-2-oxo-chinolin-3-carboxamid.

**32.** Verwendung nach einem der Ansprüche 1 - 3 und 5 von Verbindungen, worin $R_5$ ausgewählt ist aus Methyl, Ethyl, Methoxy, Ethoxy, Chlor und Brom oder $R_5$ und $R_6$ zusammengenommen Methylendioxy sind.

**33.** Verwendung nach einem der Ansprüche 1 - 3, 5 und 32 von Verbindungen, worin R ausgewählt ist aus Methyl, Ethyl, n-Propyl.

**34.** Verwendung nach einem der Ansprüche 1 - 3, 5 und 32 von Verbindungen, worin R Wasserstoff ist.

**35.** Verwendung nach einem der Ansprüche 1 - 3, 5 und 32 von Verbindungen, worin R Methyl ist und R' Wasserstoff ist.

**36.** Verwendung nach einem der Ansprüche 1 - 3, 5 und 32 von Verbindungen, worin $R_{42}$ und $R_{43}$ Wasserstoff sind.

**37.** Verwendung nach einem der Ansprüche 1 - 3, 5 und 32 von Verbindungen, worin R' ausgewählt ist aus Wasserstoff, para-Methyl, -Methoxy, -Chlor und -Trifluormethyl.

**38.** Verwendung nach einem der Ansprüche 1 - 3, 5 und 32 von Verbindungen, worin R" ausgewählt ist aus meta'- und para-Fluor, mit der Maßgabe, dass R' ortho-Fluor ist.

**39.** Verwendung nach Anspruch 5 der Verbindung N-Methyl-N-pnenyl-4-amino-1,2-dihydro-1,5-dimethyl-2-oxo-chinolin-3-carboxamid.

**40.** Verwendung nach Anspruch 5 der Verbindung N-Methyl-N-phenyl-4-amino-1,2-dihydre-5-methoxy-1-methyl-2-oxo-chinolin-3-carboxamid.

**41.** Verwendung nach Anspruch 5 der Verbindung N-Methyl-N-phenyl-1,2-dihydro-1,5-dimethyl-4-(N-methylamino)-2-oxo-chinolin-3-carboxamid.

**42.** Verwendung nach Anspruch 5 der Verbindung N-Methyl-N-phenyl-1,2-dihydro-5-methoxy-4-(N-methylamino)-1-methyl-2-oxo-chinolin-3-carboxamid.

**43.** Verwendung nach Anspruch 5 der Verbindung N-Methyl-N-(4-trifluormethyl)phenyl-4-amino-1,2-dihydro-1-methyl-2-oxo-chinolin-3-carboxamid.

**44.** Verbindung der Formel (I)

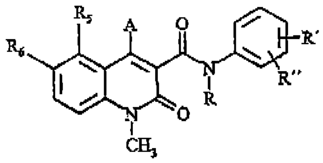

(I)

worin
A $NR_{42}R_{43}$ ist, worin
$R_{42}$ und $R_{43}$ gleich oder voneinander verschieden sind und ausgewählt sind aus Wasserstoff, Methyl, Ethyl, n-Propyl, Isopropyl und Cyclopropyl; oder
$R_{42}$ $COR_B$ ist, worin
$R_B$ $C_1$-$C_4$-Alkyl ist, oder
$COR_B$ eine 2-Acyloxymethylbenzoylgruppe ist

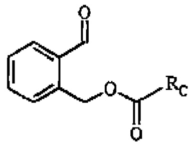

worin $R_C$ ausgewählt ist aus Methyl, Ethyl, Phenyl und Benzyl;
R ausgewählt ist aus Wasserstoff, Methyl, Ethyl, n-Propyl, Isopropyl, n-Butyl, Isobutyl, sec-Butyl und Allyl;
R' ausgewählt ist aus Wasserstoff, Methyl, Methoxy, Fluor, Chlor, Brom, Cyano, Trifluormethyl und $OCH_xF_y$, worin

$$x = 0 - 2,$$

$$y = 1 - 3$$

mit der Maßgabe, dass

$$x + y = 3;$$

R" ausgewählt ist aus Wasserstoff, Fluor und Chlor, mit der Maßgabe, dass R" nur aus Fluor und Chlor ausgewählt ist, wenn R' aus Fluor und Chlor ausgewählt ist;
$R_5$ ausgewählt ist aus Wasserstoff, Methyl, Ethyl, n-Propyl, Isopropyl, Methoxy, Ethoxy, Fluor, Chlor, Brom, Trifluormethyl, Dimethylamino und $OCH_xF_y$, und $OCH_2CH_xF_y$,
worin

$$x = 0 - 2,$$

$$y = 1 - 3$$

mit der Maßgabe, dass

$$x + y = 3$$

und

mit der Maßgabe, dass $R_5$ nur Wasserstoff ist, wenn R' Trifluormethyl ist;
$R_6$ Wasserstoff ist; oder
$R_5$ und $R_6$ zusammengenommen Methylendioxy sind;
oder ein beliebiges Tautomer davon.

**45.** Verbindung nach Anspruch 44, worin $R_5$ ausgewählt ist aus Methyl, Ethyl, Methoxy, Ethoxy oder $R_5$ und $R_6$ zusammengenommen Methylendioxy sind.

**46.** Verbindung nach Anspruch 44 oder 45, worin R ausgewählt ist aus Methyl, Ethyl und n-Propyl.

**47.** Verbindung nach Anspruch 44 oder 45, worin R Wasserstoff ist.

**48.** Verbindung nach Anspruch 44 oder 45, worin R Methyl ist und R' Wasserstoff ist.

**49.** Verbindung nach Anspruch 44 oder 45, worin $R_{42}$ und $R_{43}$ Wasserstoff sind

**50.** Verbindung nach Anspruch 44 oder 45, worin R' ausgewählt ist aus Wasserstoff und Trifluormethyl.

**51.** Verbindung nach Anspruch 44, welche N-Methyl-N-phenyl-4-amino-1,2-dihydro-1,5-dimethyl-2-oxo-chinolin-3-carboxamid ist.

**52.** Verbindung nach Anspruch 44, welche N-Methyl-N-phenyl-4-amino-1,2-dihydro-5-methoxy-1-methyl-2-oxo-chinolin-3-carboxamid ist.

**53.** Verbindung nach Anspruch 44, welche N-Methyl-N-phenyl-1,2-dihydro-1,5-dimethyl-4-(N-methylamino)-2-oxo-chinolin-3-carboxamid ist.

**54.** Verbindung nach Anspruch 44, welche N-Methyl-N-phenyl-1,2-dihydro-5-methoxy-4-(N-methylamino)-1-methyl-2-oxo-chinolin-3-carboxamid ist.

**55.** Verbindung nach Anspruch 44, welche N-Methyl-N-(4-trifluormethyl)phenyl-4-amino-1,2-dihydro-1-methyl-2-oxo-chinolin-3-carboxamid ist.

**56.** Verbindung N-Ethyl-N-phenyl-1,2-dihydro-4-hydroxy-5-dimethylamino-1-methyl-2-oxo-chinolin-3-carboxamid.

**57.** Verbindung N-Methyl-N-(4-methoxy-phenyl)-1,2-dihydro-5-dimethylamino-4-hydroxy-1-methyl-2-oxo-chinolin-3-carboxamid.

**58.** Verbindung nach einem der Ansprüche 44 bis 57, die als Therapeutikum verwendet werden soll.

**59.** Pharmazeutische Zusammensetzung, umfassend als Wirkstoff eine Verbindung nach einem der Ansprüche 44 bis 57 zusammen mit einem pharmazeutisch annehmbaren Träger.

**60.** Pharmazeutische Zusammensetzungen nach Anspruch 59, umfassend andere therapeutisch aktive Substanzen.

**61.** Pharmazeutische Zusammensetzungen nach Anspruch 59 in einer Dosierungsform, die ausreicht, um eine Tagesdosis der aktiven Substanz von 0,002 mg/kg bis ungefähr 100 mg/kg Körpergewicht, insbesondere zwischen 0,02 mg/kg bis 10 mg/kg Körpergewicht bereitzustellen.

**62.** Verfahren zur Herstellung einer Verbindung nach einem der Ansprüche 44 bis 55 durch Reaktion eines 4-Chlor-1,2-dihydro-2-oxo-chinolin-3-carboxamid-derivats der Formel (II)

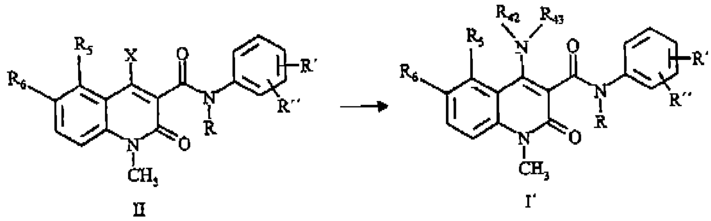

mit einer Aminoverbindung in einem geeigneten Alkohol, z. B. Ethanol.

## Revendications

**1.** Utilisation de composés de formule générale (I)

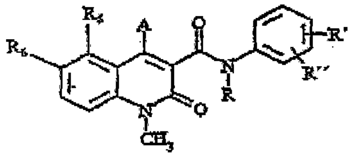

(I)

dans laquelle
A est choisi parmi $OR_{41}$ et $NR_{42}R_{43}$ où
$R_{41}$ est choisi parmi l'hydrogène, les cations inorganiques pharmaceutiquement acceptables, le sodium, le potassium et le calcium ; les cations organiques, la monoéthanolamine, la diéthanolamine, le diméthylaminoéthanol et la morpholine ; et $COR_A$ où
$R_A$ est choisi parmi le méthyle, l'éthyle, le n-propyle, l'isopropyle, le tert-butyle, le néopentyle, le phényle, le benzyle et-le phénéthyle ;
$R_{42}$ et $R_{43}$ sont identiques ou différents et choisis parmi l'hydrogène, le méthyle, l'éthyle, le n-propyle, l'isopropyle et le cyclopropyle ; ou
$R_{42}$ est $COR_B$ où
$R_B$ est un alkyle en C1 à C4, ou
$COR_B$ est un groupe 2-acyloxyméthylbenzoyle

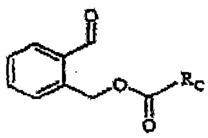

où $R_C$ est choisi parmi le méthyle, l'éthyle, le phényle et le benzyle ;
R est choisi parmi l'hydrogène, le méthyle, l'éthyle, le n-propyle, l'isopropyle, le n-butyle, l'isobutyle, le sec-butyle et l'allyle ; à condition que R ne soit pas un hydrogène lorsque A est $OR_{41}$;
R' est choisi parmi l'hydrogène, le méthyle, le méthoxy, le fluoro, le chloro, le bromo, le cyano, le trifluorométhyle et $OCH_xF_y$,
où

$$x = 0 \text{ à } 2,$$

$$y = 1 \text{ à } 3$$

à condition que

$$x + y = 3 ;$$

et à condition que R’ ne soit pas un hydrogène lorsque R est un méthyle et A est $OR_{41}$ ;
R" est choisi parmi l’hydrogène, le fluoro et le chloro, à condition que R" soit choisi parmi le fluoro et le chloro seulement lorsque R’ est choisi parmi le fluoro et le chloro ;
$R_5$ est choisi parmi l’hydrogène, le méthyle, l’éthyle, le n-propyle, l’isopropyle, le méthoxy, l’éthoxy, le fluoro, le chloro, le bromo, le trifluorométhyle, le diméthylamino, et $OCH_xF_y$ et $OCH_2CH_xF_y$,
où

$$x = 0 \text{ à } 2,$$

$$y = 1 \text{ à } 3$$

à condition que

$$x + y = 3$$

et
à condition également que $R_5$ ne soit pas un fluoro lorsque A est $OR_{41}$ ; et que $R_5$ soit un hydrogène uniquement lorsque A est $NR_{42}R_{43}$ et R’ est un trifluorométhyle ;
$R_6$ est un hydrogène ; ou
$R_5$ et $R_6$ pris ensemble sont un méthylènedioxy ;
ou un quelconque tautomère de ceux-ci ;
pour la fabrication d’un médicament pour le traitement d’une maladie tumorale maligne solide.

**2.** Utilisation selon la revendication 1, dans laquelle la maladie tumorale maligne solide est choisie parmi les cancers du sein, les cancers du côlon, le sarcome de Kaposi, les cancers du poumon, les cancers de l’ovaire, les cancers de la prostate et les cancers de la peau.

**3.** Utilisation selon la revendication 2, dans laquelle la maladie tumorale maligne solide est un cancer du sein ou un cancer de la prostate.

**4.** Utilisation selon l’une quelconque des revendications 1 à 3 de composés dans lesquels A est $OR_{41}$.

**5.** Utilisation selon l’une quelconque des revendications 1 à 3 de composés dans lesquels A est $NR_{42}R_{43}$.

**6.** Utilisation selon l’une quelconque des revendications 1 à 4 de composés dans lesquels $R_5$ est un méthyle, un éthyle, un méthoxy, un chloro, un bromo, ou $R_5$ et $R_6$ pris ensemble sont un méthylènedioxy.

**7.** Utilisation selon l’une quelconque des revendications 1 à 4 et 6 de composés dans lesquels R est un méthyle et un éthyle.

**8.** Utilisation selon l’une quelconque des revendications 1 à 4 et 7 de composés dans lesquels R est un éthyle et R’ est un hydrogène.

**9.** Utilisation selon l’une quelconque des revendications 1 à 4 et 6 de composés dans lesquels R’ est choisi parmi le para-méthyle, -méthoxy, -chloro, -trifluorométhyle.

**10.** Utilisation selon l’une quelconque des revendications 1 à 4 et 6 de composés dans lesquels R" est choisi parmi le méta’- et para-fluoro à condition que R’ soit un ortho-fluoro.

**11.** Utilisation selon la revendication 4 de composés dans lesquels $R_{41}$ est choisi parmi l'hydrogène et le sodium.

**12.** Utilisation selon la revendication 4 du composé N-éthyl-N-phényl-1,2-dihydro-4-hydroxy-1,5-diméthyl-2-oxo-quinoléine-3-carboxamide.

**13.** Utilisation selon la revendication 4 du composé N-éthyl-N-phényl-1,2-dihydro-4-hydroxy-5-éthyl-1-méthyl-2-oxo-quinoléine-3-carboxamide.

**14.** Utilisation selon la revendication 4 du composé N-éthyl-N-phényl-1,2-dihydro-4-hydroxy-5-méthoxy-1-méthyl-2-oxo-quinoléine-3-carboxamide.

**15.** Utilisation selon la revendication 4 du composé N-éthyl-N-phényl-1,2-dihydro-4-hydroxy-5-chloro-1-méthyl-2-oxo-quinoléine-3-carboxamide ou de son sel de sodium.

**16.** Utilisation selon la revendication 4 du composé N-éthyl-N-phényl-1,2-dihydro-4-hydroxy-5-bromo-1-méthyl-2-oxo-quinoléine-3-carboxamide.

**17.** Utilisation selon la revendication 4 du composé N-éthyl-N-phényl-1,2-dihydro-4-hydroxy-5,6-méthylènedioxy-1-inéthyl-2-oxo-quinoléine-3-carboxamide.

**18.** Utilisation selon la revendication 4 du composé N-éthyl-N-phényl-1,2-dihydro-4-hydroxy-5-diméthylamino-l-méthyl-2-oxo-quinoléine-3-carboxamide.

**19.** Utilisation selon la revendication 4 du composé N-éthyl-N-(3-fluorophényl)-1,2-dihydro-4-hydroxy-5-chloro-1-méthyl-2-oxo-quinoléine-3-carboxamide.

**20.** Utilisation selon la revendication 4 du composé N-méthyl-N-(4-chlorophényl)-1,2-dihydro-4-hydroxy-5-chloro-1-méthyl-2-oxo-quinoléine-3-carboxamide.

**21.** Utilisation selon la revendication 4 du composé N-méthyl-N-(4-méthylphényl)-1,2-dihydro-4-hydroxy-5-chloro-1-méthyl-2-oxo-quinoléine-3-carboxamide.

**22.** Utilisation selon la revendication 4 du composé N-méthyl-N-(2,4-difluorophényl)-1,2-dihydro-4-hydroxy-5-chloro-1-méthyl-2-oxo-quinoléine-3-carboxamide.

**23.** Utilisation selon la revendication 4 du composé N-méthyl-N-(2,5-difluorophényl)-1,2-dihydro-4-hydroxy-5-chloro-1-méthyl-2-oxo-quinoléine-3-carboxamide.

**24.** Utilisation selon la revendication 4 du composé N-éthyl-N-(3-méthoxyphényl)-1,2-dihydro-4-hydroxy-5-éthyl-1-méthyl-2-oxo-quinoléine-3-carboxamide.

**25.** Utilisation selon la revendication 4 du composé N-éthyl-N-(4-chlorophényl)-1,2-dihydro-4-hydroxy-1,5-diméthyl-2-oxo-quinoléine-3-carboxamide.

**26.** Utilisation selon la revendication 4 du composé N-méthyl-N-(2,4-difluorophényl)-1,2-dihydro-4-hydroxy-5-éthyl-1-méthyl-2-oxo-quinoléine-3-carboxamide.

**27.** Utilisation selon la revendication 4 du composé N-méthyl-N-(2,4-difluorophényl)-1,2-dihydro-4-hydroxy-5-méthoxy-1-méthyl-2-oxo-quinoléine-3-carboxamide.

**28.** Utilisation selon la revendication 4 du composé N-méthyl-N-(2,5-difluorophényl)-1,2-dihydro-4-hydroxy-5-méthoxy-1-méthyl-2-oxo-quinoléine-3-carboxamide.

**29.** Utilisation selon la revendication 4 du composé N-méthyl-N-(4-trifluorométhylphényl)-1,2-dihydro-4-hydroxy-5-méthoxy-1-méthyl-2-oxo-quinoléine-3-carboxamide.

**30.** Utilisation selon la revendication 4 du composé N-méthyl-N-(2,4-difluorophényl)-1,2-dihydro-4-hydroxy-5,6-méthylenedioxy-1-méthyl-2-oxo-quinoléine-3-carboxamide.

**31.** Utilisation selon la revendication 4 du composé N-méthyl-N-(4-méthoxyphényl)-1,2-dihydro-5-diméthylamino-4-hydroxy-1-méthyl-2-oxo-quinoléine-3-carboxamide.

**32.** Utilisation selon l'une quelconque des revendications 1 à 3 et 5 de composés dans lesquels $R_5$ est choisi parmi le méthyle, l'éthyle, le méthoxy, l'éthoxy, le chloro et le bromo, ou $R_5$ et $R_6$ pris ensemble sont un méthylènedioxy.

**33.** Utilisation selon l'une quelconque des revendications 1 à 3, 5 et 32 de composés dans lesquels R est choisi parmi le méthyle, l'éthyle, le n-propyle.

**34.** Utilisation selon l'une quelconque des revendications 1 à 3, 5 et 32 de composés dans lesquels R est un hydrogène.

**35.** Utilisation selon l'une quelconque des revendications 1 à 3, 5 et 32 de composés dans lesquels R est un méthyle et R' est un hydrogène.

**36.** Utilisation selon l'une quelconque des revendications 1 à 3, 5 et 32 de composés dans lesquels $R_{42}$ et $R_{43}$ sont un hydrogène.

**37.** Utilisation selon l'une quelconque des revendications 1 à 3, 5 et 32 de composés dans lesquels R' est choisi parmi l'hydrogène, le para-méthyle, -méthoxy, -chloro et -trifluorométhyle.

**38.** Utilisation selon l'une quelconque des revendications 1 à 3, 5 et 32 de composés dans lesquels R" est choisi parmi le méta'- et para-fluoro à condition que R' soit un ortho-fluoro.

**39.** Utilisation selon la revendication 5 du composé N-méthyl-N-phényl-4-amino-1,2-dihydro-1,5-diméthyl-2-oxo-quinoléine-3-carboxamide.

**40.** Utilisation selon la revendication 5 du composé N-méthyl-N-phényl-4-amino-1,2-dihydro-5-méthoxy-1-méthyl-2-oxo-quinoléine-3-carboxamide.

**41.** Utilisation selon la revendication 5 du composé N-méthyl-N-phényl-1,2-dihydro-1,5-diméthyl-4-(N-méthylamino)-2-oxo-quinoléine-3-carboxamide.

**42.** Utilisation selon la revendication 5 du composé N-méthyl-N-phényl-1,2-dihydro-5-méthoxy-4-(N-méthylamino)-1-méthyl-2-oxo-quinoléine-3-carboxamide.

**43.** Utilisation selon la revendication 5 du composé N-méthyl-N-(4-trifluorométhyl)phényl-4-amino-1,2-dihydro-1-méthyl-2,-oxo-quinoléine-3-carboxamide.

**44.** Composé de formule (I)

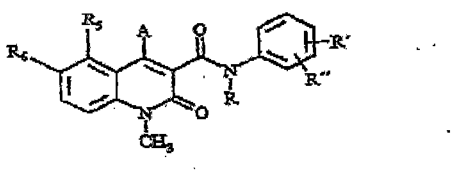

(I)

dans laquelle
A est $NR_{42}R_{43}$ où
$R_{42}$ et $R_{43}$ sont identiques ou différents et choisis parmi l'hydrogène, le méthyle, l'éthyle, le n-propyle, l'isopropyle et le cyclopropyle ; ou
$R_{42}$ est $COR_B$ où
$R_B$ est un alkyle en C1 à C4, ou
$COR_B$ est un groupe 2-acyloxyméthylbenzoyle

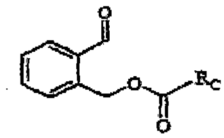

où $R_C$ est choisi parmi le méthyle, l'éthyle, le phényle et le benzyle ;
R est choisi parmi l'hydrogène, le méthyle, l'éthyle, le n-propyle, l'isopropyle, le n-butyle, l'isobutyle, le sec-butyle et l'allyle ;
R' est choisi parmi l'hydrogène, le méthyle, le méthoxy, le fluoro, le chloro, le bromo, le cyano, le trifluorométhyle et $OCH_xF_y$,
où

$$x = 0 \text{ à } 2,$$

$$y = 1 \text{ à } 3$$

à condition que

$$x + y = 3 \text{ ;}$$

R" est choisi parmi l'hydrogène, le fluoro et le chloro, à condition que R" soit choisi parmi le fluoro et le chloro seulement lorsque R' est choisi parmi le fluoro et le chloro ;
$R_5$ est choisi parmi l'hydrogène, le méthyle, l'éthyle, le n-propyle, l'isopropyle, le méthoxy, l'éthoxy, le fluoro, le chloro, le bromo, le trifluorométhyle, le diméthylamino, et $OCH_xF_y$ et $OCH_2CH_xF_y$,
où

$$x = 0 \text{ à } 2,$$

$$y = 1 \text{ à } 3$$

à condition que

$$x + y = 3$$

et
à condition également que $R_5$ soit un hydrogène uniquement lorsque R' est un trifluorométhyle ;
$R_6$ est un hydrogène ; ou
$R_5$ et $R_6$ pris ensemble sont un méthylènedioxy ;
ou un quelconque tautomère de ceux-ci.

**45.** Composé selon la revendication 44 dans lequel $R_5$ est choisi parmi le méthyle, l'éthyle, le méthoxy, l'éthoxy, ou $R_5$ et $R_6$ pris ensemble sont un méthylènedioxy.

**46.** Composé selon la revendication 44 ou 45 dans lequel R est choisi parmi le méthyle, l'éthyle et le n-propyle.

**47.** Composé selon la revendication 44 ou 45 dans lequel R est un hydrogène.

**48.** Composé selon la revendication 44 ou 45 dans lequel R est un méthyle et R' est un hydrogène.

**49.** Composé selon la revendication 44 ou 45 dans lequel $R_{42}$ et $R_{43}$ sont un hydrogène.

**50.** Composé selon la revendication 44 ou 45 dans lequel R' est choisi parmi l'hydrogène et le trifluorométhyle.

**51.** Composé selon la revendication 44 qui est le N-méthyl-N-phényl-4-amino-1,2-dihydro-1,5-diméthyl-2-oxo-quinoléine-3-carboxamide.

**52.** Composé selon la revendication 44 qui est le N-méthyl-N-phényl-4-amino-1,2-dihydro-5-méthoxy-1-méthyl-2-oxo-quinoléine-3-carboxamide.

**53.** Composé selon la revendication 44 qui est le N-méthyl-N-phényl-1,2-dihydro-1,5-diméthyl-4-(N-méthylamino)-2-oxo-quinoléine-3-carboxamide.

**54.** Composé selon la revendication 44 qui est le N-méthyl-N-phényl-1,2-dihydro-5-méthoxy-4-(N-méthylamino)-1-méthyl-2-oxo-quinoléine-3-carboxamide.

**55.** Composé selon la revendication 44 qui est le N-méthyl-N-(4-trifluorométhyl)phényl-4-amino-1,2-dihydro-1-méthyl-2-oxo-quinoléine-3-carboxamide.

**56.** composé N-éthyl-N-phényl-1,2-dihydro-4-hydroxy-5-diméthylamino-1-méthyl-2-oxo-quinoléine-3-carboxamide.

**57.** Composé N-méthyl-N-(4-méthoxyphényl)-1,2-dihydro-5-diméthylamino-4-hydroxy-1-méthyl-2-oxo-quinoléine-3-carboxamide.

**58.** Composé selon l'une quelconque des revendications 44 à 57 devant être utilisé comme thérapeutique.

**59.** Composition pharmaceutique comprenant en tant qu'ingrédient actif un composé selon l'une quelconque des revendications 44 à 57 conjointement avec un véhicule pharmaceutiquement acceptable.

**60.** Compositions pharmaceutiques selon la revendication 59 comprenant d'autres substances thérapeutiquement actives.

**61.** Compositions pharmaceutiques selon la revendication 59 sous une forme posologique suffisante pour délivrer un dosage journalier de la substance active de 0,002 mg/kg à environ 100 mg/kg de poids corporel, en particulier entre 0,02 mg/kg et 10 mg/kg de poids corporel.

**62.** Procédé de préparation d'un composé selon l'une quelconque des revendications 44 à 55 par réaction d'un dérivé du 4-chloro-1,2-dihydro-2-oxo-quinoléine-3-carboxamide de formule (II)

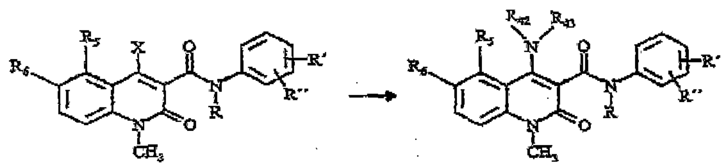

II

I'

avec un composé amino dans un alcool approprié, par exemple, l'éthanol.